(19) Europäisches Patentamt
European Patent Office
Office européen des brevets

(11) Publication number : **0 494 777 A1**

(12) **EUROPEAN PATENT APPLICATION**

(21) Application number : **92300172.1**

(51) Int. Cl.$^5$ : **C07D 221/18,** A61K 31/47

(22) Date of filing : **09.01.92**

(30) Priority : **11.01.91 US 640241**

(43) Date of publication of application :
**15.07.92 Bulletin 92/29**

(84) Designated Contracting States :
**AT BE CH DE DK ES FR GB GR IT LI LU MC NL PT SE**

(71) Applicant : **Cannon, Joseph Gerald**
**920 Highwood Street**
**Iowa City, Iowa 52246 (US)**
(71) Applicant : **Moe, Scott Thomas**
**1150 Cushing Circle, No. 219**
**St. Paul, Minnesota 55108 (US)**
(71) Applicant : **Long, John Paul**
**1817 Kathlin Drive**
**Iowa City, Iowa 52246 (US)**
(71) Applicant : **Bhatnagar, Ranbir Krishna**
**2965 E. Washington**
**Iowa City, Iowa 52246 (US)**

(72) Inventor : **Cannon, Joseph Gerald**
**920 Highwood Street**
**Iowa City, Iowa 52246 (US)**
Inventor : **Moe, Scott Thomas**
**1150 Cushing Circle, No. 219**
**St. Paul, Minnesota 55108 (US)**
Inventor : **Long, John Paul**
**1817 Kathlin Drive**
**Iowa City, Iowa 52246 (US)**
Inventor : **Bhatnagar, Ranbir Krishna**
**2965 E. Washington**
**Iowa City, Iowa 52246 (US)**

(74) Representative : **Stephenson, Gerald Frederick**
**Patents Department, British Technology Group plc, 101 Newington Causeway**
**London SE1 6BU (GB)**

(54) **(S)-11-Hydroxy-10-methyl aporphine and pharmaceutical compositions containing it.**

(57) (S)-11-Hydroxy-10-methylaporphine and its physiologically acceptable salts show activity as 5-$HT_{1A}$ inhibitors and can be used as an antidote for effects of cocaine and as appetite suppressants.

EP 0 494 777 A1

Jouve, 18, rue Saint-Denis, 75001 PARIS

This invention relates to compounds of value as 5-$HT_{1A}$ receptor inhibitors and to compositions containing them.

Numerous 5-$HT_{1A}$ receptor inhibitors are known but none of those so far discovered is selective. Thus, those known at present are often also blockers of other receptors such as dopamine, norepinephrine and/or acetyl choline and, in particular, none is selective only for serotonin 5-$HT_{1A}$ receptor blocking.

It is highly desirable to have as a blocker a compound which functions very specifically for the blocking of serotonin receptors since this allows highly specific effects to be achieved without accompanying undesirable side effects. The applications for a highly specific serotonin 5-$HT_{1A}$ receptor blocker include use as an antidote for counteracting the effects of cocaine and as an appetitite suppressant.

Cannon et al, Journal of Medicinal Chemistry, 1988, 31, 313-318, report that (R)-11-hydroxy-10-methylaporphine is an agonist for serotonin 5-$HT_{1A}$ receptors. It has now surprisingly been found that the (S) isomer of the same compound not only shows no agonist effect but will block the action of serotonin 5-$HT_{1A}$ receptors, i.e. it is an antagonist. This is therefore one of the very rare examples of enantiomers which demonstrate exactly opposite pharmacological effects at the same receptor.

Accordingly the present invention comprises (S)-11-hydroxy-10-methylaporphine and physiologically acceptable salts thereof.

(S)-11-Hydroxy-10-methylaporphine has the following formula:

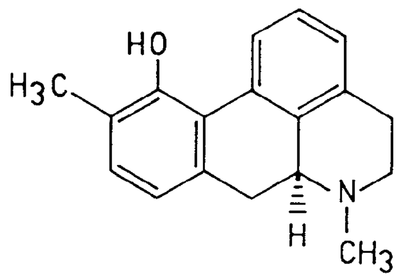

As already indicated, Cannon et al., Journal of Medicinal Chemistry, 31, 313-318 (1988) describe the preparation of the corresponding (R) enantiomer of the compound of the present invention and its properties as an agonist for serotonin 5-$HT_{1A}$ receptors. The compound of the present invention is an antagonist for serotonin 5-$HT_{1A}$ receptors and is of value for inhibiting 5-$HT_{1A}$ neuroreceptors in a mammal, for example a human. Such a method can be useful for two purposes, namely as an antidote for the effects of cocaine and for appetite suppression. The compound of the present invention is of particular value in that it is a selective serotonin 5-$HT_{1A}$ neuroreceptor inhibitor and does not inhibit different neurotransmitter receptors such as dopamine and acetyl choline. It will be appreciated from the foregoing discussion that it is the (S) enantiomer which has the valuable properties and this invention does not extend to the (RS) racemate of 11-hydroxy-10-methylaporphine and its physiologically acceptable salts. Nevertheless, although the (S) enantiomer is ideally free of the (R) enantiomer, some degree of contamination of the (S) enantiomer by the (R) enantiomer can be accepted. It is preferred, however, that the amount of the (R) enantiomer [in either the (R) or the (RS) form] contained in a sample of the (S) enantiomer of the invention is less than 2 or 1% by weight, particularly less than 0.2 or 0.1% and especially less than 0.02 or 0.01% (these figures apply to the total of the (R) and (RS) forms of the compound relative to the (S) form, calculated in relation to the free bases, although the different forms of the compound can be in the free base or salt form).

As indicated, the (S)-11-hydroxy-10-methylaporphine may be used in the form of a physiologically acceptable salt, in particular an acid addition salt formed with an inorganic acid such as hydrochloric acid, hydrobromic acid, sulphuric acid, nitric acid, phosphoric acid, etc., or with an organic acid such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, etc. These physiologically acceptable salts, for example the hydrochloride, provide a preferred form of the compound for pharmaceutical use.

Administration of a therapeutically effective amount of (S)-11-hydroxy-10-methylaporphine can be effected via any of the accepted modes of administration for systemically active substances. These methods include oral, parenteral (subcutaneous, intramuscular, intravenous) and rectal administration, as well as other systemic means of administration such as the use of an aerosol.

The compound of the present invention and its salts may be formulated as a pharmaceutical composition with a physiologically acceptable diluent or carrier in various forms known in the pharmaceutical art. Such forms include tablets, pills, capsules, powders, liquids for parenteral and oral administration including oils, aqueous suspensions, solutions and emulsions, and suppositories for rectal administration. The composition may also

take the form of a long acting injectable preparation or sustained release device.

When the composition is of a solid form, solid pharmaceutical carriers such as starch, sugar, talc, mannitol, povidone, magnesium stearate, and the like may be used to form powders. Lactose and mannose are the preferred solid carriers. The powders may be used as such for direct administration to a patient or, alternatively, the powders may be added to a suitable solid or liquid foodstuff and also to other liquids, including water, to facilitate administration.

The powders may also be used to make tablets, or to fill gelatin capsules. Suitable lubricants such as magnesium stearate, binders such as gelatin, and disintegrating agents such as sodium carbonate in combination with citric acid may be used to form the tablets.

Unit dosage forms such as tablets, pills and capsules may contain any suitable predetermined amount of (S)-11-hydroxy-10-methylaporphine or particularly a physiologically acceptable acid addition salt thereof, and may be administered one or more at a time at regular intervals as appropriate. The compound of the invention or its salt may be administered at varying dosage levels but a level of from 1.0 to 25.0 mg/kg of body weight, preferably from 5.0 to 20.0 mg/kg is often suitable, repeated at intervals as required. It will be appreciated that extended administration of regular doses, possibly at a lower level, are in general more appropriate to use as an appetite suppressant whereas use as an antidote for cocaine may require higher levels, possibly over a shorter period.

The preferred method of synthesis of the (S)-11-hydroxy-10-methylaporphine enantiomer involves a modification of the previously published preparation of the (R) enantiomer, (Cannon et al., Journal of Medicinal Chemistry, 31, 313-318, 1988) and can be represented by the following Scheme, the following examples further illustrating the synthesis of the S-enantiomer according to this Scheme.

It will be seen that the final stage in the preparation of (S)-11-hydroxy-10-methylaporphine or a physiologically acceptable salt thereof as indicated in the Scheme comprises reducing (S)-10-formyl-11-hydroxyaporphine or a salt thereof, for example using hydrogenation over a Pd/C catalyst, and thereafter, where appropriate, converting the product to the desired salt form.

As an alternative to the procedure indicated in the Scheme, the racemate (RS)-11-hydroxy-10-methylaporphine or a salt thereof may be prepared, for example by reducing (RS)-10-formyl-11-hydroxy-aporphine or a salt therof, and the (S) enantiomer separated from the (R) enantiomer and, where appropriate, converted to the desired salt form.

Scheme 1. Synthesis of (*S*)-(+)-11-hydroxy-10-methylaporphine

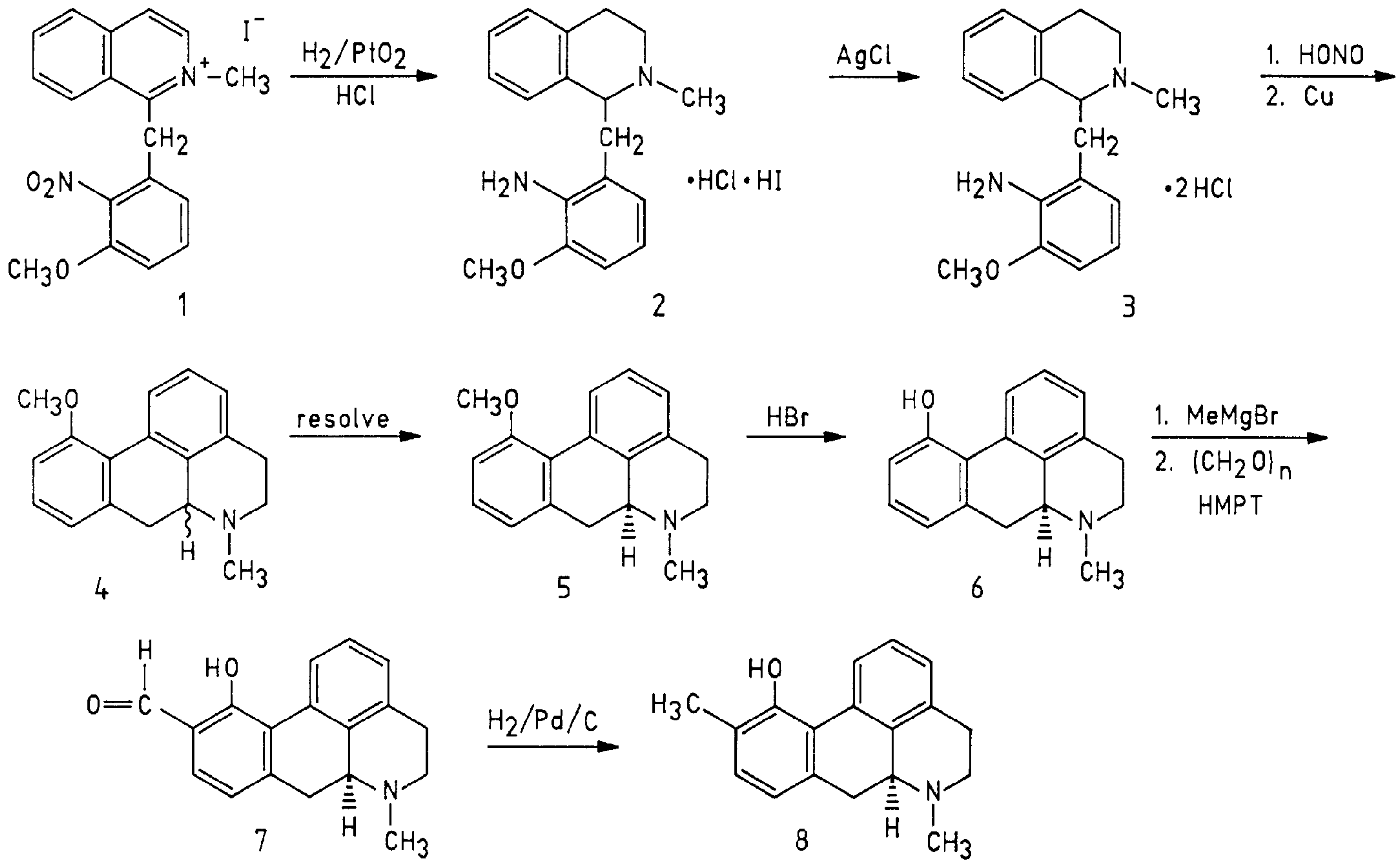


1 2 3 4 5 6 7 8

The invention is illustrated by the following Examples (the numbers after the names of the compounds in the subtitles of Example 1 corresponding to the numbering system used in the above Scheme). All reactions were conducted under $N_2$ unless otherwise indicated.

EXAMPLES

Example 1 : Preparation of (S)-11-hydroxy-10-methylaporphine hydrochloride

(1) 1-(2-Amino-3-methoxybenzyl)-2-methyl-1,2,3,4-tetrahydro-isoquinoline hydrochloride/hydriodide (2).

1-(3-Methoxy-2-nitrobenzyl)isoquino1ine methiodide (1) (12.7 g, 0.029 mol), prepared as described by Neumeyer et al., Journal of Medicinal Chemistry, 1974, 17, 1090-1095, in a mixture of 325 ml of MeOH, 100 ml of EtOH and 2 ml of $CHCl_3$ was hydrogenated in a Parr apparatus at 25°C over 0.50 g of $PtO_2$ for 48 hours at an initial pressure of 60 psig. An excess of HCl was then bubbled through the hydrogenation mixture and it was filtered through Celite. Volatile components were removed from the filtrate under reduced pressure to give the title compound (12.4 g, 96%) as an orange semi-solid which was used in the next step without purification. A small portion was crystallized twice from MeOH-EtOAc (2:3) to give the pure hydrochloride/hydroiodide, m.p. 210-212°C; MS m/e 283 ($M^+$-HCl-HI).

(2) 1-(2-Amino-3-methoxybenzyl)-2-methyl-1,2,3,4-tetrahydro-isoquinoline dihydrochloride (3).

A mixture of 12.1 g (0.0273 mol) of the hydrochloride/hydroiodide (2) and 5.0 g (0.0349 mol) of AgCl in 250 ml of MeOH was shaken on a mechanical shaker for 18 hours. The silver salts were removed by filtration through Celite and the filtrate was evaporated under reduced pressure. The solid residue was dissolved in 50 ml of methanol/isopropanol (MeOH/i-PrOH) (4:1) and the dihydrochloride salt was precipitated by the addition of 400 ml of EtOAc. The solid was collected on a filter and dried under $N_2$ to yield the title compound (9.5 g, 98%), m.p. 212-215°C. Spectral (NMR, MS) data on this salt were identical with those obtained with the HCl-HI salt. The material was used in the next step without purification.

(3) (R,S)-11-Methoxyaporphine hydrochloride (4).

The dihydrochloride (3) (5.85 g, 0.065 mol) in 500 ml of 10% $H_2SO_4$ was cooled to -5°C in an ice-salt bath and a solution of 1.50 g (0.0217 mol) of $NaNO_2$ in 25 ml of cold $H_2O$ was added dropwise over 5 minutes to the rapidly stirred solution. The reaction mixture was stirred at -5°C for 15 minutes, and then 2.0 g (0.315 g.atom) of freshly prepared Cu (vide infra) was added in one portion. The ice-salt bath was replaced with an ice-$H_2O$ bath and the reaction mixture was stirred for 18 hours allowing the ice gradually to melt. The resulting mixture was passed through a fritted glass filter and the filtrate was made basic with concentrated $NH_4OH$, then extracted with five 100 ml portions of $CHCl_3$. The pooled extracts were dried ($Na_2SO_4$) and volatile components were removed under reduced pressure. The resulting dark orange oil was dissolved in 20 ml of i-PrOH and an excess of ethereal HCl was added followed by 600 ml of anhydrous $Et_2O$. The title compound precipitated as a light orange solid (3.79, 74%), m.p. 215-220°C, which was used in the next step without purification. A small portion was recrystallized twice from EtOH-$Et_2O$ to give the pure hydrochloride as a white solid, m.p. 258-261°C (decomp); MS: m/e 266 ($M^+$-HCl).

Footnote : Preparation of Copper for Pschorr cyclization

A variation of the procedure of Gatterman, Untersuchungen uber Diazoverbindungen. 8er. Deutsch. Chem. Ges., 1890, 23, 1218-1228, was used. Zn dust (6.0 g, 0.092 g.atom; washed with two 50 ml portions of HCl followed by two 50 ml portions of 1120) was added in 10 portions over 45 minutes to a rapidly stirred solution of 20.0 g (0.0125 mol) of $CuSO_4.5H_2O$ in 100 ml of $H_2O$ at such a rate as to maintain the temperature at 20-25°C. The mixture was stirred for a further 1 hour, then the aqueous layer was decanted and the solid was stirred for 1 hour with 100 ml of 5% HCl. The copper was then washed with $H_2O$ until the washings were neutral to pH paper.

(4) (S)-11-Methoxyaporphine hydrochloride (5).

The racemic hydrochloride (4) (4.9 g, 0.0072 mol) in 15 ml of EtOAc was treated with 1.48 g (3.66 mmol)

of (+)-di-p-toluoyl-D-tartaric acid in 15 ml of EtOAc. The mixture was heated under reflux for 1 hour, then it was cooled and filtered and the solid residue was washed with two 10 ml portions of EtOAc. The resulting material was recrystallized four times from EtOAc-EtOH (1:4) to constant optical rotation. The product was dissolved in a mixture of 25 ml of $CHCl_3$, 25 ml of $H_2O$, and 1 ml of EtOH. The solution was made basic with 25 ml of saturated $NaHCO_3$ and the aqueous solution was extracted with three 25 ml portions of $CHCl_3$. The pooled organic extracts were dried ($Na_2SO_4$) and the volatile components were removed under reduced pressure. The residue was treated with ethereal HCl to give the title compound (0.29 g, 13%) as white crystals, m.p. 257-259°C (decomp);
$[\alpha]_D^{25}$ + 94.3° (c 0.51, MeOH), $[\alpha]_{578}^{25}$ + 98.3° (c 0.51, MeOH).

Footnote

The NMR and MS data for (S)-11-methoxyaporphine hydrochloride were identical with those of the (RS) modification (6) and of the (R)-enantiomer (8), which latter product can be obtained in an exactly similar procedure using (-)-di-p-toluoyl-L-tartaric acid.

(5) (S)-11-Hydroxyaporphine hydrochloride (6).

A solution of 0.300 g (1.13 mmol) of the (S)-hydrochloride (5) in 10 ml of 48% HBr was heated at 125°C for 4 hours. The cooled reaction mixture was filtered and the residue was washed with two 5 ml portions of $Me_2CO$-$Et_2O$ (3:1) to give a white solid, $[\alpha]_D^{25}$ + 64.7° (c 0.49, MeOH), $[\alpha]_{578}^{25}$ + 69.7° (c 0.49, MeOH). This material was treated with saturated $NaHCO_3$ and the aqueous solution was extracted with three 50 ml portions of $CHCl_3$. The pooled organic extracts were dried ($Na_2SO_4$) and the volatile components were taken to dryness under reduced pressure to produce the free base of the title compound (0.228 g, 91%), $[\alpha]_D^{25}$ + 115° (c 0.51, MeOH), $[\alpha]_{578}^{25}$ + 115° (c 0.51, MeOH). A portion of this material was converted into the HCl salt with ethereal HCl, the product being crystallized from EtOH-MeOH-$Et_2O$ to produce the title compound as a white solid,
m.p. 179-181°C (decomp); $[\alpha]_D^{25}$ + 75.4° (c 0.45, MeOH),
$[\alpha]_{578}^{25}$ + 73.2° (c 0.45, MeOH).

(6) (S)-10-Formyl-11-hydroxyaporphine hydrochloride (7).

This reaction was performed in a dry box under $N_2$ with 10% relative humidity. A suspension of 0.228 g (0.908 mmol) of the hydrochloride (6) in 10 ml of benzene was added in 0.5 ml portions over 20 minutes to a solution of 0.45 ml (1.36 mmol) of MeMgBr (3.0 M in $Et_2O$) in 10 ml of benzene. The mixture was stirred at room temperature for 30 minutes and a solution of 0.243 g (1.36 mmol) of hexamethylphosphorous triamide (HMPT) in 1 ml of benzene was added. The mixture was stirred for a further 15 minutes, then a suspension of 0.273 g (9.08 mmol) of paraformaldehyde in 2 ml of benzene was added. The resulting mixture was heated under reflux for 4 hours, cooled, and transferred to a 1 litre beaker. 200 ml of 5% HCl was added and this mixture was stirred for 10 minutes. The mixture was made basic with solid $NaHCO_3$ and was then extracted with 100 ml of $Et_2O$, followed by four 75 ml portions of $CHCl_3$. The combined organic extracts were dried ($Na_2SO_4$) and the volatile components were removed under reduced pressure to produce a green oil which was converted to the HCl salt with ethereal HCl. The product was washed with two 5 ml portions of absolute EtOH to give the title compound (0.130 g, 46%) as a white solid,
m.p. 255-258°C (decomp).

(7) (S)-11-Hydroxy-10-methylaporphine hydrochloride (8).

A solution of 0.120 g (0.429 mmol) of the hydrochloride (7) in 50 ml of MeOH-$CHCl_3$ (1:1) was hydrogenated at 50°C for 48 hours over 0.050 g of 10% Pd/C at an initial pressure of 50 psig. The cooled reaction mixture was filtered through Celite and the filtrate was evaporated under reduced pressure. The residue was treated with $Et_2O$ to produce a white precipitate which was recrystallized from EtOH-$Et_2O$ to give the title compound (0.056 g, 43%) as a white solid, m.p. 268-270°C (decomp);
$[\alpha]_D^{25}$ + 101° (c 0.54, MeOH), $[\alpha]_{578}^{25}$ + 104° (c 0.54, MeOH).

Example 2 : Formulation of (S)-11-hydroxy-10-methyoporphine

A typical tablet formulation has the composition (A) or (B):
(A)

| | Mg. |
|---|---|
| (S)-11-Hydroxy-10-methylaporphine | 1-25 |
| Mannitol | 100 |
| Stearic acid | 3 |

A dry granulated material is made from the mannitol and the other ingredients are added to this material and the tablets are then punched.
(B)

| | Mg. |
|---|---|
| (S)-11-Hydroxy-10-methylaporphine | 1-25 |
| Starch U.S.P. | 57 |
| Lactose U.S.P. | 73 |
| Talc U.S.P. | 9 |
| Stearic acid | 6 |

Powders of the first three ingredients are slugged, then granulated, mixed with the last two ingredients and the whole tableted.

A typical capsule preparation is obtained by filling No. 3 hard gelatin capsules with the following thoroughly mixed ingredients:

| | Mg. |
|---|---|
| (S)-11-Hydroxy-10-methylaporphine | 1-25 |
| Lactose U.S.P. | 200 |
| Starch U.S.P. | 16 |
| Talc U.S.P. | |

Example 3 : Tests on the pharmacoloqical activity of the (S)-11-hydroxy-10-methylaporphine hydrochloride

In these studies the corresponding (R) enantiomer (Cannon et al., ibid) was also included by way of comparison.

(1) Studies using rat cortex

Binding studies to determine the affinity of both the (R) and (S) enantiomer for 5-$HT_{1A}$ sites were conducted as previously described by Cannon et al., ibid. Rat cortex was homogenized in ice cold solution, centrifuged to isolate the membranes, and then washed. The final assay mixture included 50 mM Tris buffer (pH 7.5), 5% (w/v) homogenized rat cortex, 10 μM pargyline, 0.1% ascorbic acid, 4 mM $CaCl_2$, 1 nM [$^3$H]8-OH DPAT (8-hydroxy-2-di-n-propylaminotetralin), and appropriate concentrations of the (S) or (R) enantiomer. Non-specific binding was defined as binding remaining in the presence of 10 μM of 5-HT. $K_i$ values were determined by weighted non-linear least square curve fitting with a LIGAND program using a KD value for [$^3$H]8-OH-DPAT of 1.9 nM obtained from saturation curves. The results are presented below in the Table together with those obtained in the studies using guinea pig ilea.

(2) Studies using guinea pig ilea.

Guinea pigs were anaesthetized with 35 mg of pentobarbital Na, administered i.p. and then sacrificed. Two centimeters of ileum, 10 cm from the cecum, was placed in Krebs-Ringer solution, and longitudinal muscle contractions were measured using a Stateham GT-03 force transducer. Contractions were recorded using a Beckman R-611 recorder. The contractions were induced using transmural single shock stimulation (0.1 Hz), and were inhibited by $10^{-8}$ M atropine sulphate. After stabilization of the contractions, the (S) and (R) enantiomers were tested for their ability to inhibit contractions or to antagonize the inhibitory action of 8-OH DPAT (5-$HT_{1A}$ receptor agonists can inhibit muscle contractions by approximately 35% in this preparation).

The results are presented below in the Table together with those obtained in the studies using rat cortex.

Table

Biological Properties of Enantiomers of 11-Hydroxy-10-methylaporphine

| Absolute configuration | Radioligand binding constant vs. 8-OH DPAT $K_i$ (nM) | $ED_{50}$ (μM) for for antagonism of inhibition of contraction by 8-OH DPAT(1) | Single Shock stimulation of guinea pig ileum $ED_{50}$ (μM) for inhibition of contractions |
|---|---|---|---|
| (R) | 3.1 | inactive | 0.05 (0.01-0.1) |
| (S) | 39.0 | 0.03 (0.01-0.08) | inactive |

(1) Concentration of 8-OH-DPAT used was 0.06 μM. This concentration produced maximal inhibition of contractions induced by transmural stimulation which were approximately 35%.

The Table shows the qualitatively similar activity for the two enantiomers in the rat cortex binding studies but their opposite activity in the studies using guinea pig ilea. Both enantiomers are potent in their ability to displace [$^3$H] 8-OH DPAT from membranes of rat forebrain. This experimental procedure does not indicate umabiguously whether a compound is an agonist or an antagonist at the binding sites for 8-OH DPAT. However, the functional studies using guinea pig ilea demonstrate clearly the opposing actions of the two enantiomers. The (R)-enantiomer is a potent inhibitor of contractions induced by single shock stimulation of cholinergic neurons and this inhibition is antagonized by the (S)-enantiomer. The (S)-enantiomer also antagonized inhibition induced by 8-OH DPAT, but it did not facilitate nor inhibit contractions induced by field stimulations. The above results were also obtained in the presence of $10^{-6}$ M prazosin so there is no evidence for involvement of $a_1$ adrenoceptors in the responses described above.

Two or three washings following inhibition of contractions by the (R)-enantiomer or 8-OH DPAT returned responses to induced elecrical stimulation to control levels. The (S)-enantiomer was difficult to remove from the preparation and repeated washings over 2-3 hours were required to re-establish the sensitivity of the preparation to the (R)-enantiomer or to 8-OH DPAT. The (S)-enantiomer (1 μM) did not alter the resting tone of the ileum, nor did it alter response to electrical stimulation. The (S)-enantiomer (1 μM) did not alter the ileum stimulating properties of potassium chloride or of nicotine. Thus, there is no evidence that the (S)-enantiomer is acting as a smooth muscle depressant.

The unexpected phenomenon of opposite pharmacological effects (agonism-antagonism) exhibited by enantiomers has therefore been demonstrated at two different neurotransmitter receptors (dopamine/serotonin). Moreover, since serotonin 5-$HT_{1A}$ inhibition is known to be effective in counteracting the effects of cocaine and to be involved with appetite suppression, the use of the (S)-11-hydroxy-10-methylaporphine and its physiologically acceptable salts for these purposes is indicated by the pharmacological data presented herein.

## Claims

1. (S)-11-Hydroxy-10-methylaporphine and physiologically acceptable salts thereof.

2. (S)-11-Hydroxy-10-methylaporphine hydrochloride.

3. A compound according to Claim 1 or 2 which contains less than 1% by weight of the (R) and (RS) forms of the compound.

4. A compound according to Claim 3 which contains less than 0.1% by weight of the (R) and (RS) forms of the compound.

5. A pharmaceutical composition comprising (S)-11-hydroxy-10-methylaporphine or a physiologically acceptable salt thereof together with a physiologically acceptable diluent or carrier.

6. A pharmaceutical composition according to Claim 5, in which the (S)-11-hydroxy-10-methylaporphine is as defined in any of Claims 2 to 4.

7. A composition according to Claim 5 or 6 which is in injectable form.

8. A composition according to Claim 5 or 6 which is in an oral dosage form.

9. A composition according to Claim 8 which is in tablet, pill or capsule form.

10. A composition according to Claim 8 which is in a liquid form.

11. A composition according to any of Claims 5 to 10 in unit dosage form.

12. A composition comprising (S)-11-hydroxy-10-methylaporphine or a physiologically acceptable salt thereof and a foodstuff.

13. A process for the preparation of (S)-11-hydroxy-10-methylaporphine or a physiologically acceptable salt thereof which comprises either reducing (S)-10-formyl-11-hydroxyaporphine or a salt thereof or resolving the racemate (RS)-11-hydroxy-10-methylaporphine or a salt thereof to separate the (S) enantiomer from the (R) enantiomer and thereafter in either case, where appropriate, converting the product to the desired salt form.

14. A process according to Claim 13, in which (S)-10-formyl-11-hydroxyaporphine or a salt thereof is reduced.

15. A process according to Claim 13 or 14, in which the (S)-11-hydroxy-10-methylaporphine is as defined in any of Claims 2 to 4.

16. A compound being (S)-11-hydroxy-10-methylaporphine or a physiologically acceptable salt thereof for use in therapy.

17. A compound according to Claim 16, in which the (S)-11-hydroxy-10-methylaporphine is as defined in any of Claims 2 to 4.

18. The use of (S)-11-hydroxy-10-methylaporphine or a physiologically acceptable salt thereof for the manufacture of a medicament for use as an antidote for cocaine or as an appetite suppressant.

19. The use according to Claim 18, in which the (S)-11-hydroxy-10-methylaporphine is as defined in any of Claims 2 to 4.

**Claims for the following Contracting States: GR, ES**

1. A process for the preparation of (S)-11-hydroxy-10-methylaporphine or a physiologically acceptable salt thereof which comprises either reducing (S)-10-formyl-11-hydroxyaporphine or a salt thereof or resolving the racemate (RS)-11-hydroxy-10-methylaporphine or a salt thereof to separate the (S) enantiomer from the (R) enantiomer and thereafter in either case, where appropriate, converting the product to the desired salt form.

2. A process according to Claim 1, in which (S)-10-formyl-11-hydroxyaporphine or a salt thereof is reduced.

3. A process according to Claim 1 or 2, in which (S)-11-hydroxy-10-methylaporphine hydrochloride is prepared.

4. A process according to any of Claims 1 to 3, in which the (S)-11-hydroxymethylaporphine or its salt contains less than 1% by weight of the (R) and (RS) forms of the compound.

5. A process according to Claim 4, in which the (S)-11-hydroxymethylaporphine or its salt contains less than 0.1% by weight of the (R) and (RS) forms of the compound.

6. A compound being (S)-11-hydroxy-10-methylaporphine or a physiologically acceptable salt thereof for use in therapy.

7. A compound according to Claim 6, in which the (S)-11-hydroxy-10-methylaporphine is as defined in any of Claims 3 to 5.

8. The use of (S)-11-hydroxy-10-methylaporphine or a physiologically acceptable salt thereof for the manufacture of a medicament for use as an antidote for cocaine or as an appetite suppressant.

9. The use according to Claim 1, in which the (S)-11-hydroxy-10-methylaporphine is as defined in any of Claims 3 to 5.

European Patent Office

# EUROPEAN SEARCH REPORT

Application Number

EP 92 30 0172

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | JOURNAL OF MEDICINAL CHEMISTRY. vol. 31, no. 2, February 1988, WASHINGTON US pages 313 - 318; JOSEPH G. CANNON ET AL: '(R)-(-)-10-Methyl-11-hydroxyaporphine:a highly selective serotonergic agonist' * the whole document * --- | 1,2, 13-15 | C07D221/18 A61K31/47 |
| P,X | CHEMICAL ABSTRACTS, vol. 115, no. 9, 2 September 1991, Columbus, Ohio, US; abstract no. 92658Q, JOSEPH G. CANNON ET AL: 'Enantiomers of 11-hydroxy-10-methylaporphine having opposing pharmacological effects at 5-HT1a receptors' page 805 ; * abstract * & CHIRALITY vol. 3, no. 1, 1991, pages 19 - 23; ----- | 1-19 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 19 MARCH 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)